Europäisches Patentamt

European Patent Office

Office européen des brevets

(11) Veröffentlichungsnummer: **0 207 884**

**A2**

(12) ## EUROPÄISCHE PATENTANMELDUNG

(21) Anmeldenummer: 86730103.8

(51) Int. Cl.⁴: **A61B 17/58**

(22) Anmeldetag: 04.07.86

(30) Priorität: 05.07.85 DE 8519854 U

(43) Veröffentlichungstag der Anmeldung:
07.01.87 Patentblatt 87/02

(84) Benannte Vertragsstaaten:
CH DE FR GB IT LI

(71) Anmelder: **MECRON MEDIZINISCHE PRODUKTE GMBH**
**Nunsdorfer Ring 25-27**
**D-1000 Berlin 48(DE)**

(72) Erfinder: **Anapliotis, Emmanuel**
**Kiebitzweg 5**
**D-1000 Berlin 33(DE)**

(74) Vertreter: **Christiansen, Henning, Dipl.-Ing.**
**Dietrich-Schäfer-Weg 21**
**D-1000 Berlin 41(DE)**

(54) Selbstspannende gerade Knochenplatte.

(57) Selbstspannende gerade Knochenplatte, die außer Drucklöchern auch Rundlöcher enthält, wobei jeweils ein Rundloch näher zum Ende der Platte hin angeordnet ist als die beiden innersten Drucklöcher unterschiedlicher Gleitrichtung.

EP 0 207 884 A2

## Selbstspannende gerade Knochenplatte

Die Erfindung betrifft eine Knochenplatte der im Oberbegriff des Anspruchs 1 angegebenen Art.

Selbstspannende gerade Knochenplatten sind beispielsweise aus dem Aufsatz von Bagby in "The Journal of Bone and Joint Surgery", Nr. 5, Juli 1977, Seiten 625 bis 629 bekannt.

Derartige selbstspannende Druckplatten weisen Drucklöcher auf, die -in Längsrichtung gesehen - zur Mitte der Platte hin abgeschrägte Gleitbahnen aufweisen, so daß eine Schraube, die in ein derartiges Loch eingeführt wird, mit zunehmendem Anziehen die Tendenz hat, sich auf der Gleitbahn zur Plattenmitte hin zu bewegen. Da die Schraube benachbart zu einer zu fixierenden Fraktur in einen Knochen ein geschraubt wird, wird dieser mit in Richtung der gedachten Mittelachse der Platte bewegt, so daß die Fraktur auf diese Weise geschlossen wird.

Die "Mittelachse" der Platte bildet dabei diejenige Linie, welche Bereiche der Platte voneinander trennt, in denen die Drucklöcher unterschiedlich geneigte Gleitbahnen aufweisen. Diese Mittelachse ist im Bereich der Fraktur anzubringen, so daß die Gleitrichtungen der Drucklöcher beidseitig dieser Fraktur so gerichtet sind, daß sich die mit den Schrauben verbundenen Frakturelemente in Richtung auf diese gedachte Mittelachse zu bewegen. - (Bei unsymmetrischer Gestaltung der Platte braucht sich diese "Mittelachse" sich also nicht tatsächlich (bezogen auf die Längsachse der Platte) genau in der Mitte zu befinden.

Es ist bekannt, bei derartigen Druckplatten neben mit Gleitbahnen versehenen Drucklöchern auch Rundlöcher anzubringen. Die Anordnung der Rundlöcher erfolgte dabei aber stets so, daß auf der einen Seite der Mittellinie ausschließlich Rundlöcher vorgesehen waren, während Drucklö cher nur auf der anderen Seite angeordnet sind - (vergleiche den vorstehend genannten Aufsatz). Diese Anordnung bezweckte, das Vorgehen beim Befestigen von geraden Platten und Winkelplatten in Übereinstimmung zu bringen. Da auf derjenigen Seite, auf denen die Rundlöcher vorgesehen sind, kein Gleitvorgang stattfinden kann, ist der Kompressionsweg für die Fraktur verringert. Weil über jedes der Drucklöcher lediglich ein Kompressionsweg von rund einem Millimeter maximal erreicht werden kann, bedeutet dieser Verzicht einen Nachteil, da die Anwendbarkeit der Platte zunimmt, wenn der Kompressionsweg größer ist.

Andererseits besteht bei denjenigen Platten, welche ausschließlich Drucklöcher aufweisen, der Nachteil, daß bei Resorption von Knochenmaterial im Bereich der Fraktur die Fixtionswirkung einer derartigen Platte nachläßt, weil die Kompressionswirkung ausschließlich in Richtung auf die Fraktur besteht -also nur eine Auseinanderbewegung der Frakturteile parallel zur Plattenrichtung bezogen auf die Ausgangssitution gesperrt ist. Eine Platte, welche nicht in der Lage ist, die Konstanz im Fixationsbereich aufrechtzuerhalten, stellt aber eine Gefahr dar, weil Mikrobewegungen das Verheilen der Fraktur verhindern können. Ziel einer derartigen Plattenosteosynthese ist es ja gerade, die Fraktur so ruhig zu stellen, daß beschleunigt ein Gefäßaufbau und Verheilen stattfinden kann. Bewegungen im Mikrobereich sind dabei äußerst - schädlich und können den Heilungsverlauf durch Erzeugung von Pseudarthrosen im schlechtesten Fall vollständig unterbinden.

Der im Anspruch 1 angegebenen Erfindung liegt die Aufgabe zugrunde, eine Platte der eingangs genannten Gattung so auszubilden, daß sie einerseits über ihre Drucklöcher einen maximalen Kompressionsweg zuläßt, während andererseits die erreichte Kompression gegen nachträgliche Verschiebungen gesichert ist, so daß eine unerwünschte Beweglichkeit der Platte gegenüber dem zu fixierenden Knochen verhindert ist.

Die Erfindung beruht auf der Erkenntnis, daß die nachträgliche Beweglichkeit einer ausschließlich mit Drucklöc ern ausgestatteten Platte - welche bisher sogar gelegentlich als Vorteil angesehen wurde -äußerst schädlich ist. Zwar gestattet diese Beweglichkeit ein "Setzen" der Fraktur, wenn der gebrochene Knochen ausschließlich unter Druckbelastung steht, so daß ein Verheilen nur dann unbehindert stattfinden kannt, wenn außer einem gleichmäßigen Druck keine weiteren Kräfte auf die Fraktur ausgeüb werden. Da die Plattenosteosynthese aber dazu dient, den Patienten möglichst frühzeitig eine -unter Berücksichtigung der Schonung der Fraktur -eingeschränkte Bewegungsmöglichkeit zurückzugeben, ist damit zu rechnen, daß auch Torsions-oder Biegebeanspruchungen auftreten. Dabei spielt eine besondere Rolle, daß die Platte den Knochen nur einseitig — tützt und wenn sie einer Biegebeanspruchung ausgesetzt ist der Frakturbereich entweder aufklafft oder die Tendenz hat sich in dem Sinn zu - schließen, daß nur die von der Platte entfernt gelegenen Bereiche der Fraktur aufeinandergepreßt werden und dann möglicherweise unter der eintretenden werden und dann möglicherweise unter der eintretenden Überlastung nachgeben, so daß sich der Frakturspalt in diesem Bereich zusätzlich erweitert.

Bei einer entsprechenden Wechsellast werden also die beiden Teile der Fraktur bei einer Biegung der Platte nach außen und der Verwendung von ausschließlich Drucklöchern aufeinander zu wandern, wobei die benachbarten plattennahen Bereiche des Knochens nur mit geringem Querschnitt aufeinander liegen und bei einer Druckbelastung gegebenenfalls nachgeben, so daß ein weiteres Nachrutschen der Frakturteile des Knochens die Folge ist.

Bei einer Biegung der Platte in Gegenrichtung werden die betreffenden Frakturteile dann aber wieder voneinander entfernt, so daß ein Verrutschen der Knochenfragmente bezüglich der Platte in entgegengesetzter Richtung -also wieder zurück -erfolgen wird. Eine derartige Bewegungsfolge setzt sich bei Auftreten der Wechsellast -unter fortwährender Vergrößerung des möglichen Verschiebungswegs - fort , so daß die Fraktur nicht in der notwendigen Weise ruhiggestellt ist.

Durch die erfindungsgemäßen Maßnahmen werden diese Nachteile sicher vermieden.

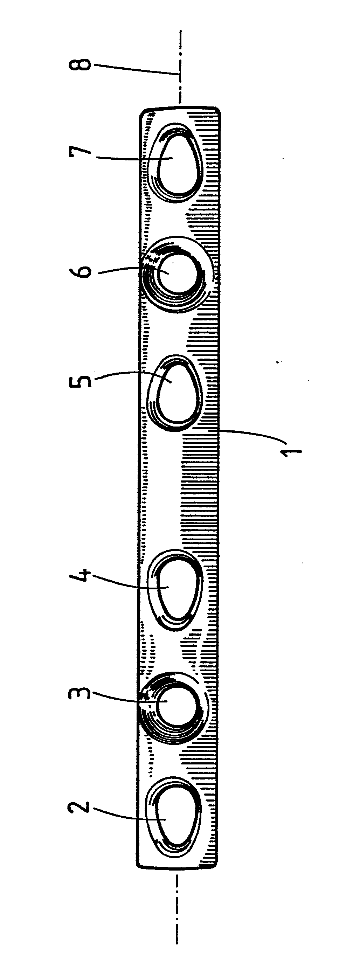

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Zeichnung näher dargestellt.

Die einzige Figur zeigt ein Ausführungsbeispiel der erfindungsgemäßen Knochenplatte in perspektivischer Draufsicht.

Bei der in der Figur dargestellten Platte handelt es sich um eine gerade Knochenplatte, welche eine gewisse Wölbung in Querrichtung aufweist, so daß sie sich an lange Röhrenknochen in Längsrichtung anschmiegt. In Längsrichtung der Platte sind sechs Löcher 2 bis 7 hintereinander angeordnet, wobei die Löcher 2, 4, 5 und 7 als Drucklöcher ausgebildet sind, welche Abschrägungen aufweisen, die beim Eindrehen von Knochenschrauben die Schraube in Richtung zur Symmetrieachse 8 der Platte hin wandern lassen. Die Schrauben 3 und 6 sind als Rundlöcher ausgebildet, d.h. beim Einschrauben und Festziehen von Knochenschrauben durch diese Bohrungen hindurch wird keine Verschiebung von Schraube und Platte relativ zueinander erzeugt. Es wird im Gegenteil eine in Längsrichtung nicht verschiebliche Verbindung bewirkt.

Es ist ersichtlich, daß bei der erfindungsgemäßen Platte gemäß Ausführungsbeispiel die runden Bohrungen so angeordnet sind, daß zwischen diesen runden Bohrungen mindestens eine Druckbohrung jeder Gleitrichtung vorhanden ist, die Rundbohrungen also mindestens um eine Druckbohrung vom vorgesehenen Frakturbereich des mit der Platte zu fixierenden Knochens enfernt gelegen sind.

Zur Fixierung des Knochens mit der erfindungsgemäßen Druckplatte werden zunächst die Bohrungen, welche dem Frakturspalt am nächsten gelegen sind -und Drucklöcher aufweisen -, mit Schrauben versehen und diese so festgezogen, daß sich die angestrebte Gleitwirkung von Knochen und Platte relativ zueinander ergibt, welche den Frakturspalt verkleinert. Anschließend kann mit dem Festziehen von weiteren Schrauben bezüglich der anderen Druckbohrungen 2 und 7 eine weitergehende Kompression des Fraktur spaltes erreicht werden, wobei die Langlöcher 4 und 5 eine zusätzliche Beweglichkeit der Schraubenköpfe zu Symmetrieachse 8 hin über die abgeschrägte Gleitbahn hinaus zulassen.

Ist der Frakturspalt auf diese Weise möglichst weitgehend geschlossen, werden die Schrauben in die Rundbohrungen 3 und 6 eingebracht, welche ein weiteres Verschieben der Knochensegmente verhindern, so daß insbesondere bei in bezug auf die Platte eintretenden Biegebelastungen keine alternierenden Verschiebebewegungen eintreten können.

Das erfindungsgemäße Ausführungsbeispiel stellt lediglich eine mögliche Realisierung der Erfindung dar. Die Rundbohrungen 3 und 6 können bevorzugt auch als letzte Bohrung in der Reihe der Löcher angeordnet sein, so daß über die zwischen ihnen liegenden Drucklöcher auch mehrere Frakturspalte (bei Mehrfachbrüchen) geschlossen werden können. Entsprechend den zu erfüllenden Forderungen kann die Anordnung der Löcher auch unsymmetrisch gewählt werden.

## Ansprüche

1. Selbstspannende gerade Knochenplatte, die außer Drucklöchern auch Rundlöcher enthält,

**dadurch gekennzeichnet,**

daß jeweils ein Rundloch (3, 6) näher zum Ende der Platte (1) hin angeordnet ist als die beiden innersten Drucklöcher (4, 5) unterschiedlicher Gleitrichtung.

2. Knochenplatte nach Anspruch 1, **dadurch gekennzeichnet,** daß die Rundlöcher (3, 6) in der Nähe der Enden der Platte (1) vorgesehen sind, während sonst ausschließlich Drucklöcher (2, 4, 5, 7) vorhanden sind.

3. Knochenplatte nach einem der vorangehenden Ansprüche, **dadurch gekennzeichnet,** daß -von der Mitte aus in Längsrichtung gesehen -beidseitig sowohl Drucklöcher (2, 4, 5, 7) als auch mindestens je ein Rundloch (3, 6) vorgesehen ist.

4. Knochenplatte nach einem der vorangehenden Ansprüche, **dadurch gekennzeichnet,** daß das letzte bzw. vorletzte Loch (3, 6) als Rundloch ausgestaltet ist, während die übrigen Löcher Drucklöcher (2, 4, 5, 7) bilden.

5. Knochenplatte nach einem der vorangehenden Ansprüche, **dadurch gekennzeichnet,** daß die Anordnung der Löcher (2 bis 7) -von der Mitte her in Längsrichtung der Platte (1) gesehen - symmetrisch ist.

ME 35.8-EU

0 207 884